# EUROPEAN PATENT APPLICATION

(11) **EP 1 792 911 A1**
(43) Date of publication of application: **06.06.2007**
(21) Application number: 05781977.3
(22) Date of filing: 12.09.2005
(51) Int. Cl.: C07K 7/06, C07K 4/12, C07K 14/47, C07K 19/00

(54) **AMINO ACID SEQUENCE ESSENTIAL TO NEUROSPECIFIC GENE EXPRESSION**

(30) Priority: 15.09.2004 JP 2004267770
(71) Applicant: Yokohama City University, Yokohama-shi Kanagawa 236-0027 (JP)
(72) Inventor: NISHIMURA, Yoshifumi, Mitaka-shi, Tokyo 181-0013 (JP); NOMURA, Mitsuru, Yokohama-shi, Kanagawa 244-0002 (JP)
(74) Representative: Woods, Geoffrey Corlett
(86) International application number: PCT/JP2005/016704
(87) International publication number: WO 2006/030722

(57) **Abstract**

A region in NRSF/REST was specified at which it interacts with mSin3B. A peptide of 8 to 50 amino acids, comprising the following amino acid sequence: Leu-Ile-Met-Leu-Ala-Asn-Val-Ala (I).

## Description

### TECHNICAL FIELD

The present invention relates to peptides and complexes both comprising amino acid sequences essential to neuron specific gene expression.

### BACKGROUND ART

Formation of normal cells, tissues and organs in the body is achieved through expression of genes at appropriate times, in appropriate places and in appropriate quantities. As a result, normal function is exerted. For example, neural genes must be expressed properly in neuronal cells and not in non-neural cells. NRSE/RE1 (neural restrictive silencer element/repressor element 1) is a silencer consisting of 21 base pairs and present in the vicinity of neuron specific genes. This silencer plays a central role in neuron-specific transcriptional regulation of more than 30 genes for such as neurotransmitter synthetases, ion channels, neuronal growth-associated proteins, and so forth. This silencer does not work in neuronal cells and suppresses expression of neuron specific genes in non-neuronal cells, to thereby assure expression of neuron specific genes in neuronal cells. It is also believed that this silencer is not only involved in the expression control of neuron specific genes but also involved in terminal differentiation of neuronal cells. It is NRSF/REST (neural restrictive silencer factor) that has been identified as a transcriptional repression factor which binds to the above-described NRSE/RE1 and represses expression of neuron specific genes in non-neuronal cells.

Naruse et al. revealed that the N-terminal transcriptional repressor domain of NRSF/REST recruits HDAC through co-repressor mSin3 and that the C-terminal transcriptional repressor domain thereof recruits HDAC through CoREST, and suggested that NRSF/REST represses transcription by deactivation of the chromatin structure (Non-Patent Document 1).
Non-Patent Document 1: Naruse Y. et al., Proc. Natl. Acad. Sci. USA, 96, 13691-13696 (1999)

### DISCLOSURE OF THE INVENTION

### PROBLEM FOR SOLUTION BY THE INVENTION

To date, the laboratory of the present inventors has shown that an N-terminal domain of NRSF/REST interacts with the PAH1 domain of mSin3B. However, what residues of the N-terminal are specifically required for transcriptional repression has not been elucidated yet.

Under circumstances, it is an object of the present invention to specify the region on NRSF/REST that interacts with mSin3B and further to elucidate the recognition mechanism at the atomic level by carrying out structure analysis of complexes using NMR.

It is another object of the present invention to obtain novel findings about transcriptional repression by experiments using transcriptional co-repressor N-CoR (which is known to interact with mSin3B) and by structure comparison with the complex between Mad1 (a tumor suppressor whose structure is known) and the PAH2 domain of mSin3.

### MEANS TO SOLVE THE PROBLEM

The present inventors prepared several interaction candidate regions of NRSF/REST and added them individually to an aqueous solution of ¹⁵N-labeled mSin3B PAH1 domain. Then, ¹H-¹⁵N HSQC was measured to roughly determine a region required for the interaction. Subsequently, the region required for the interaction with mSin3B was specified from the results of protein-protein NOE assignment by NMR filtering. The present invention has been achieved based on these findings.

A summary of the present invention is as described below.
(1) A peptide of 8 to 50 amino acids, comprising the following amino acid sequence: Leu-Ile-Met-Leu-Ala-Asn-Val-Ala (SEQ ID NO: 1) (I)
(2) The peptide according to (1) above, wherein Gln is added to the N terminus of sequence (I) and/or Leu is added to the C terminus of sequence (I).
(3) The peptide according to (1) above, wherein Ala-Pro-Gln is added to the N terminus of sequence (I) and/or Leu-Thr is added to the C terminus of sequence (I).
(4) The peptide according to any one of (1) to (3) above, which is capable of binding to the PAH1 domain of Sin3.
(5) The peptide according to any one of (1) to (4) above, which contains an amino acid sequence identical to the amino acid sequence of a specific region of neural restrictive silencer factor NRSF/REST.
(6) The peptide according to (5) above, which contains the amino acid sequence as shown in any one of SEQ ID NO: 3, 4 or 5.
(7) The peptide according to any one of (1) to (6) above, wherein basic amino acid residues are added to its C terminus.
(8) The peptide according to (7) above, wherein an amino acid residue with an aromatic ring is further added.
(9) The peptide according to (8) above, which contains the amino acid sequence as shown in SEQ ID NO: 8.
(10) A peptide of 8 to 50 amino acids, comprising the following amino acid sequence: Φ¹-X¹-X²-Φ²-A¹-X³-A²-Ala (SEQ ID NO: 2) (II)
   where Φ¹ and Φ² each independently represent a bulky hydrophobic amino acid residue; X¹, X² and X³ each independently represent any amino acid residue other than Pro; and
   A¹ and A² are each independently represent Ala or Val.
(11) The peptide according to (10) above, which is capable of binding to the PAH1 domain of Sin3.
(12) The peptide according to (10) or (11) above, which contains an amino acid sequence identical to the amino acid sequence of a specific region of neural restrictive silencer factor NRSF/REST or transcriptional co-repressor N-CoR.
(13) The peptide according to (12) above, which contains the amino acid sequence as shown in SEQ ID NO: 3, 4, 5 or 10.
(14) The peptide according to any one of (10) to (13), wherein basic amino acid residues are added to its C terminus.
(15) The peptide according to (14), wherein an amino acid residue with an aromatic ring is further added.
(16) The peptide according to (15), which contains the amino acid sequence as shown in SEQ ID NO: 8.
(17) A complex formed by the peptide according to any one of (1) to (16) above and the PAH1 domain of Sin3.
(18) A method of screening for substances effective in preventing and/or treating neurological diseases, comprising determining whether or not a test substance affects the interaction between the peptide according to any one of (1) to (16) above and the PAH1 domain of Sin3.
(19) A method of detecting neurological diseases, comprising using the peptide according to any one of (1) to (16) above or an antibody thereto.

The term "silencer" used in the present specification refers to a cis-element for repressing transcription of genes. Like an enhancer which has transcription activating ability, a silencer works regardless of the location, distance and orientation relative to the gene it controls. Transcription is repressed by the binding of a silencer factor to a silencer. NRSE/RE1 is the first silencer identified in the nervous system which is involved in cell-specific expression regulation.

"mSin3" is one of mammal-derived scaffold proteins involved in the transcriptional regulation mechanism. Sin3 which has no tissue-specificity is expressed widely and conserved highly from yeast to human. Sin3 has four PAH (paired amphipathic helix) domains each having different partners of interaction (such as transcription factor, co-repressor or methylated-CpG-binding protein) and HDAC-interaction domains (Xie, A.Y. et al., J. Virol., 76, 11809-11818 (2002)). PAH1 domain is positioned nearest to the N-terminus among the four PAH domains and is known to interact with transcriptional co-repressor N-CoR, etc. (Heinzel T. et aL, Nature, 387, 43-48 (1997); Alland L. et al., Nature, 387, 49-55 (1997)).

In the present specification, the term "PAH1 domain of Sin3 (or mSin3)" or "Sin3 (or mSin3) PAH1 domain" is intended to include not only a peptide consisting of a naturally derived sequence but also the peptide to which other sequences (for example, an artificial sequence such as Gly-Ser-His-Met to facilitate purification when the peptide is produced in an *Escherichia coli* mass expression system) are added. mSin3 has two isoforms: mSin3A and mSin3B (Heinzel T. et al., Nature, 387, 43-48 (1997)).

"N-CoR" (nuclear receptor co-repressor) refers to a transcriptional co-repressor of approximately 270 kDa. Nuclear hormone receptors, when they are not bound to ligands, form complexes with mSin3 or HDAC through N-CoR to thereby repress transcription. A related factor highly homologous to N-CoR is SMRT.

"¹H-¹⁵N HSQC" refers to spectra showing correlation between covalently bound ¹H and ¹⁵N. With ¹H-¹⁵N HSQC, it is possible to observe the NH of the main chain amide (from which Pro and N-terminus are removed) and the NH of partial side chains. ¹H-¹⁵N HSQC serves as the most basic spectra when determining the steric structures of proteins labeled with stable isotopes or discussing the mobility of such proteins. ¹H-¹⁵N HSQC also serves as effective means to analyze interactions and is recently used in screening for candidate compounds for drug discovery.

In the Example described later, the NOE of {¹H}-¹⁵N is measured. Intensity changes in the NOE of {¹H}-¹⁵N reflect the movement of ¹H-¹⁵N vectors within the protein. Observation results reveal the dynamics of the main chain. Thus, it is possible to judge whether the region of interest is a random region with high mobility in the protein or a region which has formed a certain structure. In evaluation using peak intensity ratio of NOE/NoNOE resonances, the ratio becomes smaller when the internal movement is greater.

In the specification and drawings of the present application, the abbreviations used for bases (nucleotides) and amino acids are those recommended by the IUPAC-IUB Commission on Biochemical Nomenclature or those conventionally used in the art. Examples of such abbreviations are given below.
DNA: deoxyribonucleic acid
A: Adenine
T: Thymine
G: Guanine
C: Cytosine
Ala or A: Alanine
Val or V: Valine
Leu or L: Leucine
Ile or I: Isoleucine
Pro or P: Proline
Phe or F: Phenylalanine
Trp or W: Tryptophan
Met or M: Methionine
Gly or G: Glycine
Ser or S: Serine
Thr or T: Threonine
Cys or C: Cysteine
Gln or Q: Glutamine
Asn or N: Asparagine
Tyr or Y: Tyrosine
Lys or K: Lysine
Arg or R: Arginine
His or H: Histidine
Asp or D: Aspartic acid
Glu or E: Glutamic acid

Unless otherwise specified, nucleotide sequences are described in the direction from the 5' end to 3' end and amino acid sequences are described in the direction from the N terminus to the C terminus.

### EFFECT OF THE INVENTION

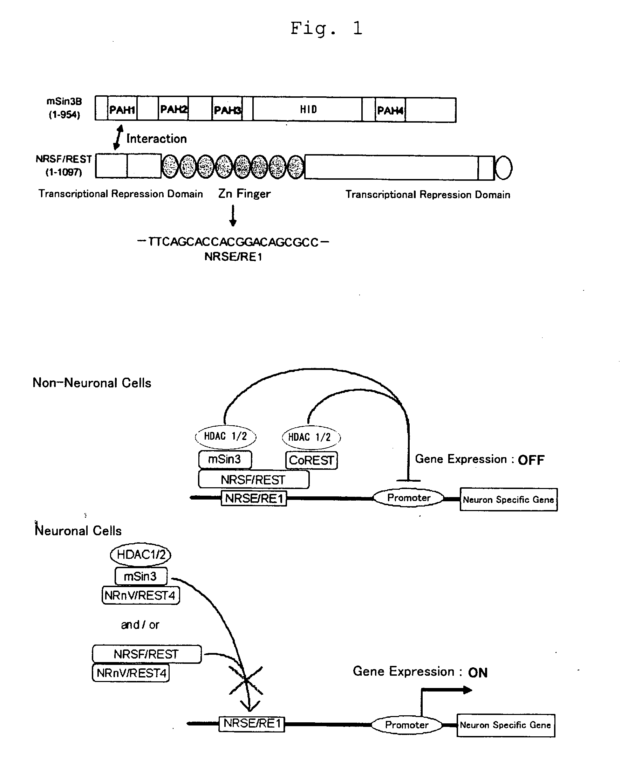

According to the present invention, amino acid sequences essential to the protein-protein interaction involved in neuronal gene expression have been found. Neural restrictive silencer factor NRSF/REST does not work in neuronal cells and suppresses expression of neuron specific genes in non-neuronal cells, to thereby assure expression of neuron specific genes in neuronal cells (Fig. 1). The interaction between NRSF/REST and co-repressor mSin3 is involved in the above-mentioned transcriptional repression mechanism. The present invention has specified amino acid sequences in NRSF/REST necessary for its interaction with mSin3.

NRSF/REST and abnormal expression of genes regulated by NRSF/REST are involved in neurodegenerative diseases (such as Down's syndrome, Alzheimer's disease and Huntington's disease) and medulloblastoma.

Down's syndrome is a disease caused by chromosome 21 trisomy mutation. Examination of difference in genes between neural tissues of embryos died from Down's syndrome and those of normal embryos revealed that expression of SCG10 (a neuron-specific growth-associated protein) gene and genes regulated by NRSF/REST decreased greatly in the former. On the other hand, those proteins regulated by transcription factors other than NRSF/REST were expressed normally (Bahn S. et al., Lancet, 359, 310-315 (2002)).

Alzheimer's disease is a disease caused by accumulation of β amyloid and neurofibrillary tangle as well as neuronal death. Expression of SCG10 was altered in Alzheimer's disease brains (Okazaki T., et al., Neurobiol Aging, 16, 883-894 (1995)).

Medulloblastoma is the most malignant brain tumor in children. Expression levels of NRSF/REST in medulloblastoma cells are very high. A recombinant protein REST-VP16 that antagonizes NRSF/REST and activates target genes thereof promotes expression of neuronal genes and activates the caspase cascade, to thereby induce apoptosis. REST-VP16 is a potential therapeutic (Lawinger P. et al., Nature Med., 7, 826-831 (2000)).

Huntington's disease is a progressive, neurodegenerative disease manifesting choreic movement, dementia and personality change as major symptoms. It is believed that abnormal huntingtin molecules with a repeat structure of glutamine residues form aggregates to thereby induce neurodegeneration. Wild-type huntingtin binds to NRSF/REST in the cytoplasm to regulate the binding of NRSF/REST to NRSE/RE1. On the other hand, this control is lost in Huntington's disease; thus, neuronal genes are not expressed sufficiently (Zuccato C. et al., Nature Genetics, 35, 76-83 (2003)).

Since NRSF/REST plays a central role in neuron-specific transcriptional regulation, this factor may be involved in neurological diseases other than those described above.

A fundamental treatment protocol has not yet been established in neurodegenerative diseases. Even in medulloblastoma for which a treatment protocol has been established, the burden on patients is very heavy because radiation on the total brain and the total spinal cord is carried out together with chemotherapy after removal of the tumor.

For the treatment of neurodegenerative diseases (such as Down's syndrome, Alzheimer's disease and Huntington's disease) and medulloblastoma, currently there are no drugs which are targeted at the interaction site of NRSF/REST with mSin3. Therefore, development of drugs with a new mode of action can be expected.

With respect to Alzheimer's disease, drugs which inhibit the generation of β amyloid (that is believed to be the major causative of this disease) or remove β amyloid are being developed. When drugs which target NRSF/REST have been developed, there is a possibility that a new method of treatment may be developed which works by a mode of action different from the mode of action of those drugs mentioned above. With respect to medulloblastoma, development of drugs which target the interaction site of NRSF/REST with mSin3 may expand choice of treatment methods other than tumor removal operation.

The specification of the present application encompasses the contents described in the specification and/or drawings of Japanese Patent Application No. 2004-267770 based on which the present application claims priority.

### BRIEF DESCRIPTION OF THE DRAWINGS

[Fig. 1] A model diagram showing expression of neuron specific genes in neuronal cells and expression repression of neuron specific genes in non-neuronal cells.
[Fig. 2] ¹H-¹⁵N HSQC of ¹⁵N-mSin3B PAH1 in the absence and in the presence of NRSF/REST₃₈₋₅₇, NRSF/REST₄₃₋₈₃, NRSF/REST₃₉₋₅₅ and NRSF/REST₄₂₋₅₆.
[Fig. 3] Chemical shift changes of mSin3B PAH1 when NRSF/REST₄₂₋₅₆ was added. mSin3B PAH1:NRSF/REST= 1:0, 1:0.5, 1:1 and 1:2 from the left.
[Fig. 4] Structure information and {¹H}-¹⁵N NOE of mSin3B PAH1 in complexes.
[Fig. 5] Superposed spectra from N-CoR titration experiment.

### BEST MODES FOR CARRYING OUT THE INVENTION

Hereinbelow, the present invention will be described in more detail.

The first invention of the present application provides a peptide of 8 to 50 amino acids, comprising the following amino acid sequence:
Leu-Ile-Met-Leu-Ala-Asn-Val-Ala (SEQ ID NO: 1) (I)

The number of amino acid residues of the peptide of the first invention of the present application is 8 to 50, preferably 10 to 40, more preferably 15 to 30.

The peptide of the first invention of the present application may further comprise Gln added to the N terminus of sequence (I) and/or Leu added to the C terminus of sequence (I). Alternatively, the peptide of the first invention of the present application may further comprise Ala-Pro-Gln added to the N terminus of sequence (I) and/or Leu-Thr added to the C terminus of sequence (I).

The peptide of the first invention of the present application may be a peptide capable of binding to the PAH1 domain of Sin3. Specific examples of Sin3 include Sin3 proteins derived from eukaryotes (e.g., yeast, *Drosophila*), derived from mammals (e.g., human, rat, mouse) and derived from amphibians (e.g., *Xenopus*). Specific examples of the PAH1 domain of Sin3 include a region of residues 31-98 in mouse-derived mSin3B (Spronk C.A. et al., Nat. Struct. Biol., 7, 1100-1104 (2000)), a region of residues 38-105 in human-derived Sin3B (Spronk C.A. et al., Nat. Struct. Biol., 7, 1100-1104 (2000)), a region of residues 114-189 in human-derived Sin3A (Brubaker K. et al., Cell, 103, 655-665 (2000)), and a region of residues 120-187 in mouse-derived mSin3A (Spronk C.A. et al., Nat. Struct. Biol., 7, 1100-1104 (2000)). The peptide of the first invention of the present application may be capable of binding to any one of these regions. The PAH1 domain of Sin3 may be a peptide consisting of a naturally derived sequence or the peptide to which other sequences (for example, an artificial sequence such as Gly-Ser-His-Met to facilitate purification when the peptide is produced in an *Escherichia coli* mass expression system) are added.

Sequence (I) is a consensus sequence found in the NRSF/REST interaction region capable of binding to the PAH1 domain of mSin3. For example, sequence (I) is found in a region of residues 46-53 of human NRSF/REST.

The peptide of the first invention of the present application may be a peptide containing an amino acid sequence identical to the amino acid sequence of a specific region of neural restrictive silencer factor NRSF/REST (e.g., a region comprising amino acid residues interacting with the PAH1 domain of Sin3). For example, peptides containing the amino acid sequences of SEQ ID NOS: 3, 4 and 5, respectively, contain amino acid sequences identical to the amino acid sequences of regions of residues 43-83, 39-55 and 38-57 of human NRSF/REST.

The peptide of the first invention of the present application may have basic amino acid residues added to its C terminus. By this addition, the solubility of the peptide can be improved. Further, an amino acid residue with an aromatic ring may be added to the peptide. This addition enables quantitative determination of concentrations using UV.

One example of the amino acid sequence of a peptide which has basic amino acid residues (Lys-Lys-Lys-Lys) added to the C-terminus of a specific region of neural restrictive silencer factor NRSF/REST (a region of residues 42-56) and has an amino acid residue with an aromatic ring (Trp) further added is shown in SEQ ID NO: 8.

The second invention of the present application provides a peptide of 8 to 50 amino acids, comprising the following amino acid sequence:
Φ¹-X¹-X²-Φ²-A¹-X³-A²-Ala (SEQ ID NO: 2) (II)
where Φ¹ and Φ² each independently represent a bulky hydrophobic amino acid residue; X¹, X² and X³ each independently represent any amino acid residue other than Pro; and A¹ and A² are each independently represent Ala or Val.

As specific examples of the "bulky hydrophobic amino acid residue" for Φ¹ and Φ², Leu, Ile, Val, Met, Phe, Trp and Tyr may be enumerated. Among them, Leu and Ile are preferred.

As specific examples of the amino acid residues for X¹, X² and X³, Ala, Met, Leu, Val, Ile, Phe, Trp, Glu, Asp, Arg, Lys, His, Gln, Asn, Ser and Thr may be enumerated. Among them, Ala, Met, Ile, Asp and Asn are preferred.

The peptide of the second invention of the present application may be a peptide capable of binding to the PAH1 domain of Sin3. Specific examples of Sin3 include Sin3 proteins derived from eukaryotes (e.g., yeast, *Drosophila*), derived from mammals (e.g., human, rat, mouse) and derived from amphibians (e.g., *Xenopus*). Specific examples of the PAH1 domain of Sin3 include a region of residues 31-98 in mouse-derived mSin3B (Spronk C.A. et al., Nat. Struct. Biol., 7, 1100-1104 (2000)), a region of residues 38-105 in human-derived Sin3B (Spronk C.A. et al., Nat. Struct. Biol., 7, 1100-1104 (2000)), a region of residues 114-189 in human-derived Sin3A (Brubaker K. et al., Cell, 103, 655-665 (2000)), and a region of residues 120-187 in mouse-derived mSin3A (Spronk C.A. et al., Nat. Struct. Biol., 7, 1100-1104 (2000)). The peptide of the second invention of the present application may be capable of binding to any one of these regions. The PAH1 domain of Sin3 may be a peptide consisting of a naturally derived sequence or the peptide to which other sequences (for example, an artificial sequence such as Gly-Ser-His-Met to facilitate purification when the peptide is produced in an *Escherichia coli* mass expression system) are added.

Sequence (II) is a consensus sequence found in the NRSF/REST interaction region and the N-CoR interaction region both capable of binding to the PAH1 domain of mSin3. For example, sequence (II) is found in a region of residues 46-53 of human NRSF/REST and in a region of residues 1837-1844 of human N-CoR.

The peptide of the second invention of the present application may have basic amino acid residues added to its C terminus. By this addition, the solubility of the peptide can be improved. Further, an amino acid residue with an aromatic ring may be added to the peptide. This addition enables quantitative determination of concentrations using UV.

One example of the amino acid sequence of a peptide which has basic amino acid residues (Lys-Lys-Lys-Lys) added to the C-terminus of a specific region of neural restrictive silencer factor NRSF/REST (a region of residues 42-56) and has an amino acid residue with an aromatic ring (Trp) further added is shown in SEQ ID NO: 8.

The peptide of the second invention of the present application may be a peptide containing an amino acid sequence identical to the amino acid sequence of a specific region of neural restrictive silencer factor NRSF/REST or transcriptional co-repressor N-CoR (e.g., a region comprising amino acid residues interacting with the PAH1 domain of Sin3). For example, peptides containing the amino acid sequences as shown in SEQ ID NOS: 3, 4 and 5, respectively, contain amino acid sequences identical to the amino acid sequences of regions of residues 43-83, 39-55 and 38-57 of human NRSF/REST. A peptide containing the amino acid sequence as shown in SEQ ID NO: 10 contains an amino acid sequence identical to the amino acid sequence of a region of residues 1834-1847 of human transcriptional co-repressor N-CoR.

The peptides of the first and the second invention of the present application may be prepared by known methods. For example, these peptides may be prepared with a commercial peptide synthesizer. Alternatively, these peptides may be produced by conventional recombinant DNA techniques.

The amino acids that compose the peptides of the first and the second invention of the present application may be in D-, L- or DL-form. However, L-form amino acids are preferred because naturally occurring amino acids are in L-form.

The peptides of the first and the second invention of the present application may be used for development of neurological disease drugs which target the NRSF/REST interaction site with Sin3. For example, abnormal expression of NRSF/REST gene and genes targeted by NRSF/REST is observed in various neurological diseases. The peptide of the present invention may be used in screening for candidate compounds for drugs which regulate the expression of these genes.

The third invention of the present application provides a complex formed by the peptide of the first or the second invention of the present application and the PAH1 domain of Sin3.

The peptides of the first and the second invention of the present application and the PAH1 domain of Sin3 are as described above.

The PAH1 domain of Sin3 may be prepared by conventional recombinant DNA techniques. The nucleotide sequence of the mouse-derived mSin3B PAH1 domain corresponds to a nucleotide sequence of positions 7-246 in SEQ ID NO: 15. The amino acid sequence of the mouse-derived mSin3B PAH1 domain is shown in SEQ ID NO: 16.

The complex of the third invention of the present application may be prepared by adding the peptide of the first or the second invention of the present application to an aqueous solution of the Sin3 PAH1 domain at room temperature. The molar ratio of the peptide of the first or the second invention of the present application and the Sin 3 PAH1 domain may be approximately 1:1. The concentration of aqueous solution of the Sin3 PAH1 domain may be 0.1 mM to 2 mM. Formation of the complex can be confirmed by NMR.

The complex of the third invention of the present application may be used for development of neurological disease drugs which target the NRSF/REST interaction site with Sin3. For example, abnormal expression of NRSF/REST gene and genes targeted by NRSF/REST is observed in various neurological diseases. The complex of the present invention may be used in screening for candidate compounds for drugs which regulate the expression of these genes.

The fourth invention of the present application provides a method of screening for substances effective in preventing and/or treating neurological diseases, comprising determining whether or not a test substance affects the interaction between the peptide of the first or the second invention of the present application and the PAH1 domain of Sin3.

The test substance may be any substance. For example, proteins, peptides, polysaccharides, oligosaccharides, monosaccharides, low molecular weight compounds, nucleic acids (DNA, RNA, oligonucleotides, mononucleotides, etc.) and so forth may be enumerated.

One example of a method for determining whether or not a test substance affects the interaction between the peptide of the first or the second invention of the present application and the PAH1 domain of Sin3 is described below. First, the interaction between the peptide of the first or the second invention of the present application and the PAH1 domain of Sin3 is measured in the presence or absence of a test substance. The interaction may be measured using techniques such as steric structure analysis at the atomic level by X-ray crystal structure analysis, nuclear magnetic resonance (NMR) or neutron diffraction; methods of observing the shapes of complexes with an electron microscope or atomic force microscope; and docking study where stable binding mode of a test substance of any structure to the steric structure of the peptide of the first or the second invention of the present application and to the steric structure of the PAH1 domain of Sin3 is simulated by computer. Next, the interaction between the peptide of the first or second invention of the present application and the PA H1 domain of Sin3 in the presence of the test substance is compared with that in the absence of the test substance. When a significant difference is observed between the interactions in the presence of the test substance and in the absence of the test substance, the test substance can be judged a substance effective for prevention and/or treatment of neurological diseases. The test substance may intensify or weaken the interaction between the peptide of the first or the second invention of the present application and the PAH1 domain of Sin3. Neurological diseases include diseases where NRSF/REST is involved. Specifically, neurodegenerative diseases such as Down's syndrome, Alzheimer's disease and Huntington's disease; medulloblastoma and the like may be enumerated.

The fifth invention of the present application provides a method of detecting neurological diseases using the peptide of the first or the second invention of the present application or an antibody thereto.

For example, it is possible to detect neurological diseases by examining the expression of Sin3 or the PAH1 domain thereof in biological samples from subjects using the peptide of the first or the second invention of the present application. Alternatively, it is possible to detect neurological diseases by examining the expression of NRSF/REST in biological samples from subjects using an antibody to the peptide of the first or the second invention of the present application.

Specific examples of the biological samples taken from subjects include neuronal tissues, non-neuronal tissues, amniotic fluid, blood and spinal fluid.

Neural restrictive silencer factor NRSF/REST is a transcriptional regulator that binds to NRSE/RE1 which plays a critical role in transcriptional regulation of neuron specific genes. In various neurological diseases, abnormal expression of NRSF/REST gene and genes targeted by NRSF/REST is observed (as described above).

When the expression of Sin3 or the PAH1 domain thereof in biological samples from subjects is examined using the peptide of the first or the second invention of the present application, the peptide may be labeled with a dye, radioactive element, enzyme or the like. For example, the peptide labeled with biotin is added to a commercial streptavidin-immobilized column to immobilize the peptide in the column using biotin-avidin interaction. Then, an extract from neuronal tissue is added to the column so that Sin3 is adsorbed onto the column. After a denaturing treatment with 6 M guanidine chloride, the expression level of Sin3 is determined. When a significant difference is observed in the expression level compared to normal values, the subject is suspected of having a neurodegenerative disease such as Down's syndrome or Alzheimer's disease.

When the expression of NRSF/REST in biological samples from subjects is examined using an antibody to the peptide of the first or the second invention of the present application, techniques such as Western blotting, immunohistochemical analysis, ELISA and the like may be used. The antibody used for this purpose may be an antibody which specifically recognizes the target protein of measurement. The antibody may be either a monoclonal antibody or a polyclonal antibody. Such antibodies may be prepared by known methods.

When the above expression is measured by Western blotting, the antibody is detected secondarily using ¹²⁵I-labeled protein A, peroxidase-linked IgG, or the like. When the expression is measured by immunohistochemical analysis, the antibody may be labeled with a fluorescent dye, ferritin, enzyme, or the like. When the expression is measured by ELISA, the antibody may be labeled with an enzyme such as peroxidase or galactosidase.

For example, when the expression level of NRSF/REST in the spinal fluid is high, the subject may be judged to have medulloblastoma. When the expression level of NRSF/REST in neuronal cell nuclei is high, the subject is suspected to have a neurological disease such as Huntington's disease.

### EXAMPLES

Hereinbelow, the present invention will be described in more detail with reference to the following Example. The Example is provided only to illustrate the present invention and not to limit the scope of the present invention.

### [Example 1]

### Materials and Methods

### 1) Sample Preparation

### a) Preparation of NRSF/REST (a.a. 43-83) and the PAH1 Domain of mSin3B (a.a. 28-107)

Based on published sequence data, cDNAs encoding NRSF/REST and mSin3B were obtained by RT-PCR (Naruse Y. et al., Proc. Natl. Acad. Sci. USA, 96, 13691-13696 (1999)). Each of the resultant cDNAs was sub-cloned into a TA cloning vector (Invitrogen). Subsequently, a fragment of NRSF/REST (a.a. 43-83) was inserted into the BamHI-EcoRI site of a GST fusion protein expression vector (pGEX-2T; Amersham Bioscience), which was transformed into *E. coli* BL21 (DE3) strain (Novagen). In a similar manner, a fragment of the PAH1 domain of mSin3B (a.a. 28-107) was inserted into the NdeI-BamHI site of a His-tag protein expression vector (pET-28a; Novagen), which was transformed into *E. coli* BL21 (DE3) strain. The *E. coli* was cultured in a liquid medium at 37 °C, followed by expression induction with isopropyl-β-D-thiogalactopyranoside (Wako Pure Chemical Industries). Then, cells were harvested and sonicated. The resultant supernatant was subjected to affinity chromatography, GST and His-tag cutting with thrombin and gel filtration chromatography in this order to thereby obtain a sample of interest. For the identification of the sample, a mass spectrometer BIFLEX or autoFLEX from Bruker Daltonics was used. The molecular weight of NRSF/REST (a.a. 43-83) is 4511 and that of the PAH1 domain of mSin3B (a.a. 28-107) is 9585. These were confirmed with corresponding m/z values. Further, ¹³C/¹⁵N- or ¹⁵N-labeled sample of the PAH1 domain of mSin3B (a.a. 28-107) was also obtained by culturing *E. coli* cells in M9 minimum medium in which ¹⁵NH₄Cl was the sole nitrogen source and/or ¹³C-glucose was the sole carbon source. Since assignment can be performed by multinuclear multidimensional NMR without no contradiction, the PAH1 domain of mSin3B (a.a. 28-107) could be confirmed.

The DNA sequence of the NRSF/REST (a.a. 43-83) fragment inserted into the BamHI-EcoRI site of pGEX-2T is shown in SEQ ID NO: 14. The sequence consisting of nucleotide positions 1-6 of SEQ ID NO: 14 (GGATCC) represents a BamHI site; the sequence consisting of nucleotide positions 7-129 represents the DNA sequence of NRSF/REST (a.a. 43-83); and the sequence consisting of nucleotide positions 131-136 (GAATTC) represents an EcoRI site. The amino acid sequence of NRSF/REST (a.a. 43-83) is shown in SEQ ID NO: 3.

The DNA sequence of the mSin3B PAH1 domain (a.a. 28-107) fragment inserted into the NdeI-BamHI site of pET-28a is shown in SEQ ID NO: 15. The sequence consisting of nucleotide positions 1-6 of SEQ ID NO: 15 (CATATG) represents a NdeI site; the sequence consisting of nucleotide positions 7-246 represents the DNA sequence of the mSin3B PAH1 domain (a.a. 28-107); and the sequence consisting of nucleotide positions 250-255 (GGATCC) represents a BamHI site. The amino acid sequence of the mSin3B PAH1 domain (a.a. 28-107) is shown in SEQ ID NO: 16.

### b) Preparation of Synthetic Peptides

NRSF/REST (a.a. 39-55), NRSF/REST (a.a. 64-80), NRSF/REST (a.a. 38-57), NRSF/REST (a.a. 68-83), NRSF/REST (a.a. 42-56) and N-CoR (a.a. 1829-1847) were synthesized by conventional peptide synthesis methods and purified. For identification of samples, Voyager-DE (Applied Biosystems) or a mass spectrometer BIFLEX or autoFLEX from Bruker Daltonics was used. Molecular weights of NRSF/REST (a.a. 39-55), NRSF/REST (a.a. 64-80), NRSF/REST (a.a. 38-57), NRSF/REST (a.a. 68-83), NRSF/REST (a.a. 42-56) and N-CoR (a.a. 1829-1847) are 1739, 1937, 2053, 1790, 2182 and 1835, respectively. These were confirmed with corresponding m/z values.

The amino acid sequences of NRSF/REST (a.a. 39-55), NRSF/REST (a.a. 64-80), NRSF/REST (a.a. 38-57), NRSF/REST (a.a. 68-83), NRSF/REST (a.a. 42-56) and N-CoR (a.a. 1829-1847) are shown in SEQ ID NOS: 4, 6, 5, 7, 8 and 17, respectively.

### 2) Interaction Experiment

Interaction candidate regions of NRSF/REST (a.a. 43-83, 39-55, 38-57, 64-80 and 68-83) were individually added to an aqueous solution of ¹⁵N-labeled mSin3B PAH1 for measuring ¹H-¹⁵N HSQC to thereby determine a region necessary for the interaction. In order to examine binding ratios, NRSF/REST (a.a. 42-56) at varied concentrations was added to ¹⁵N-labeled mSin3B PAH1 domain in phosphate buffer (pH 6.6), followed by observation of signal changes by ¹H-¹⁵N HSQC. Since NRSF/REST (a.a. 42-56) is sparingly soluble, basic residues (KKKK) were added to its C-terminus to improve the solubility. Further, in order to carry out quantitative determination of concentrations with UV, a residue having an aromatic ring (W) was also added to the peptide. In a similar manner, the interaction between ¹⁵N-labeled mSin3B PAH1 domain and N-CoR (a.a. 1829-1847) was observed by ¹H-¹⁵N HSQC. For all of the NMR measurement, Bruker Advance 700 was used.

The amino acid sequence of NRSF/REST (a.a. 42-56) to which KKKK and W are added to its C-terminus is shown in SEQ ID NO: 8.

### 3) Structure Analysis of Complexes

Structure analysis of mSin3B PAH1 domain and NRSF/REST (a.a. 38-57) was performed by multinuclear multidimensional NMR in 20 mM phosphate buffer (pH 7.5) at 293 K. Chemical shift indices were prepared from chemical shift values of mSin3B PAH1 domain and chemical shift values of random coil to predict the tendency of secondary structures. Further, {¹H}-¹⁵N NOE was measured to obtain structural information from intramolecular movement.

### Experimental Results

### 1) Specification of the NRSF/REST Interaction Region with mSin3B PAH1 Domain

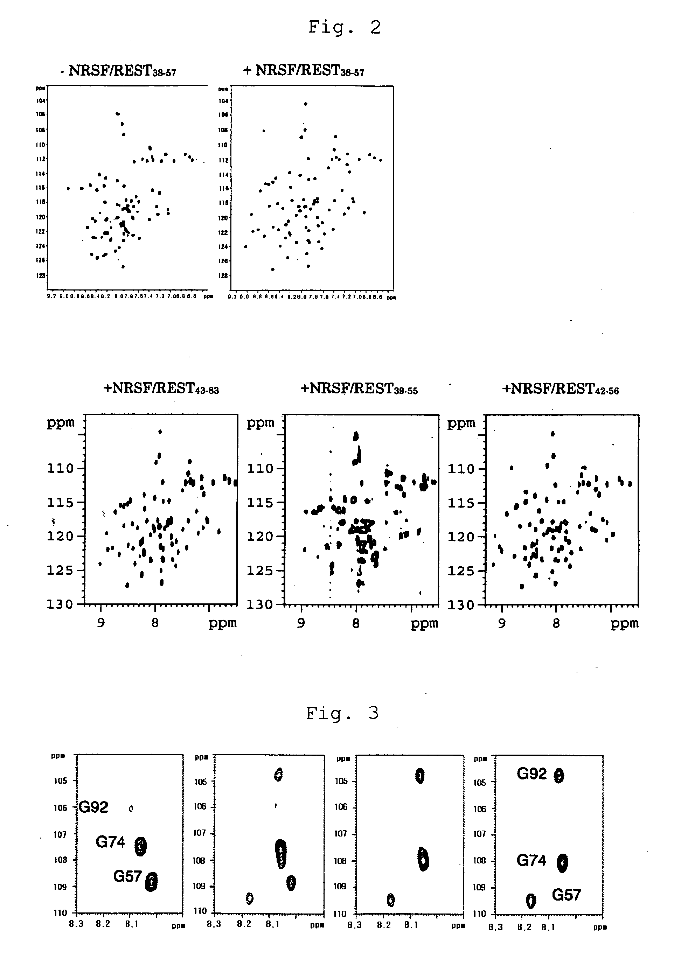

The samples with underlined residues shown in Table 1 below gave spectra of complexes as shown in the right side of Fig. 2.

**Table 1. Sequences Used in Interaction Experiment**

| NRSF/REST | | | | | Spectrum |
|---|---|---|---|---|---|
| 43-83 | | APQLIMLANVALTGEVNGSCCDYLVGEERQMAELMPVGDNN | | | Complex |
| 39-55 | AELAAPQLIMLANVALT | | | | Complex |
| 38-57 | KAELAAPQLIMLANVALTGE | | | | Complex |
| 64-80 | | | DYLVGEERQMAELMPVG | | Single peptide |
| 68-83 | | | | GEERQMAELMPVGDNN | Single peptide |
| 42-56 | | AAPQLIMLANVALTGKKKKW | | | Complex |

### 2) Titration Experiment on NRSF/REST (a.a. 42-56)

¹H-¹⁵N HSQC spectra were measured when NRSF/REST (a.a. 42-56) was added to 0.1 mM ¹⁵N-labeled mSin3B PAH1 domain at a molar ratio of 1:0, 1:0.5, 1:1 or 1:2. A part of the resultant spectra is shown in Fig. 3. As the concentration of NRSF/REST increased, signals from the single peptide mSin3B disappeared and new signals derived from the mSin3B in complex began to appear. A slow exchange process on the NMR timescale was shown. No difference was recognized between mSin3B PAH1:NRSF/REST ratios of 1:1 and 1:2. Therefore, it was shown that mSin3B PAH1 domain and NRSF/REST form a complex with 1:1.

### 3) Secondary Structure of mSin3B PAH1 Domain in Complex

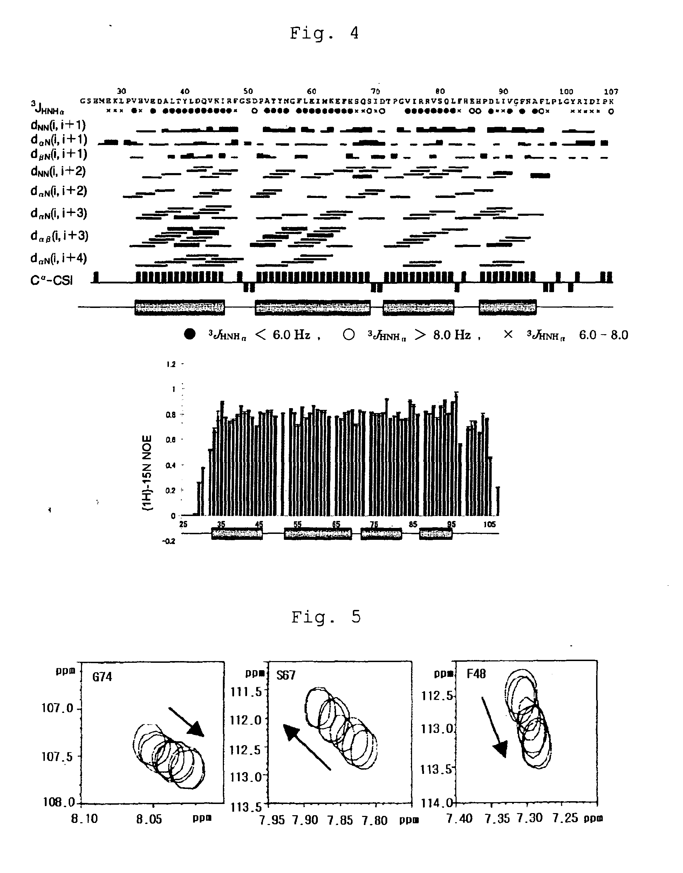

Fig. 4 shows spin-spin coupling constants ³J_{HNHα}, ¹H-¹H NOE between adjacent residues and intermediate ¹H}-¹⁵N NOE, chemical shift indices, {¹H}-¹⁵N NOE and predicted secondary structures. It was shown that PAH1 domain is composed of four α helixes. Besides, the results of {¹H}-¹⁵N NOE indicated that PAH1 domain has a low mobility region on the C-terminal side.

### 4) Interaction Experiment between Co-repressor N-CoR and mSin3B PAH1 Domain

¹H-¹⁵N HSQC spectra were measured when N-CoR was added to 0.1 mM ¹⁵N-labeled mSin3B PAH1 domain at a molar ratio of 1:0, 1:0.2, 1:0.4, 1:0.6, 1:0.8, 1:1, 1:2 or 1:3. A part of the resultant spectra superposed is shown in Fig. 5. The positions of signals shifted in a N-CoR concentration dependent manner. A rapid exchange process on the NMR timescale was shown.

### Discussion

It was found that a sample comprising NRSF/REST residues 43-55 binds to mSin3B PAH1 domain and forms a 1:1 stable complex whose structure can be analyzed by NMR. It was also found that N-CoR (a.a. 1829-1847) interacts with the PAH1 domain, though more weakly than NRSF/REST. These sequences and consensus sequences as well as the SID (Sin3-interaction domain) consensus sequences binding to PAH2 domain proposed by the group of Vuister et al. (a) and the group of Radhakrishnan et al. (b) based on the steric structure of Mad1-mSin3 PAH2 domain complex (van Ingen H. et al., Biochemistry, 43, 46-54 (2004); Swanson K.A. et al., Nat. Struct. Mol. Biol., 11, 738-746 (2004)) are shown in Table 2. Φ represents a bulky hydrophobic amino acid residue; and X represents any amino acid residue other than Proline. The SID binding to PAH1 domain and the SID binding to PAH2 domain are both composed of characteristic hydrophobic amino acids and resemble each other. However, while the second residue in the SID sequence binding to mSin3B PAH2 domain is Φ, the corresponding residue is A/V in the SID sequence binding to PAH1 domain. Further, there is no Φ on the C-terminal side of the SID sequence binding to PAH1 domain.

**Table 2. SID Consensus Sequences Binding to mSin3**

| | |
|---|---|
| NRSF/REST (a.a.43-56) | APQLIMLANVALTG |
| N-CoR (a.a.1834-1847) | ADALAALVDAAASA |
| SID binding to mSin3B PAH1 |     A A ϕXXϕ X  A V V |
| Mad1 (a.a.6-21) | RMNIQMLLEAADYLER |
| SID a) binding to mSin3B PAH2 | φφXAAXXφ |
| SID b) binding to mSin3A PAH2 | φXXφφXAA |

The amino acid sequences of NRSF/REST (a.a. 43-56), N-CoR (a.a. 1834-1847), SID binding to mSin3B PAH1, Mad1 (a.a. 6-21), SID a) binding to mSin3B PAH2 and SID b) binding to mSin3A PAH2 are shown in SEQ ID NOS: 9, 10, 2, 11, 12 and 13, respectively.

It has been shown that PAH domain is composed of four α helixes; and it has also been shown that PAH2 domain is composed of four α helixes (van Ingen H. et al., Biochemistry, 43, 46-54 (2004); Swanson K.A. et al., Nat. Struct. Mol. Biol., 11, 738-746 (2004)). Although amino acid homology between PAH1 and PAH2 is very haigh, it has been shown that the flexible loop between helixes 2 and 3 found in PAH2 domain does not exist in PAH1 domain and that PAH1 domain has a fixed loop region at its C-terminus.

As described above, individual PAH domains of mSin3 have variation in interaction, such as difference in selectivity, difference in affinity, etc. It seems that such variation allows these domains to bind to various DNA-binding transcriptional regulators or co-repressors so that necessary transcriptional repression can be achieved.

### Conclusion

1) It was confirmed that NRSF/REST (a.a. 38-57) and mSin3B PAH1 domain form a 1:1 complex.
2) From the structure analysis of the complex, it was shown that mSin3B PAH1 domain is composed of four α helixes and has a fixed loop region at its C-terminus which PAH2 domain does not have.
3) The SID consensus sequence binding to mSin3B PAH1 domain was proposed. It has been found that the recognition sequence of the SID binding to PAH1 domain is different from the recognition sequence of the SID binding to PAH2 domain.

All publications, patents and patent applications cited herein are incorporated herein by reference in their entirety.

### INDUSTRIAL APPLICABILITY

The peptide and the complex of the present invention may be used in the development of neurological disease drugs which target the NRSF/REST interaction site with Sin3.

### SEQUENCE LISTING FREE TEXT

<SEQ ID NO: 1>
   SEQ ID NO: 1 shows the amino acid sequence in NRSF/REST necessary for its interaction with mSin3.
<SEQ ID NO: 2>
   SEQ ID NO: 2 shows the consensus amino acid sequence in NRSF/REST and N-CoR necessary for their interaction with mSin3.
<SEQ ID NO: 3>
   SEQ ID NO: 3 shows the amino acid sequence of NRSF/REST residues 43-83.
<SEQ ID NO: 4>
   SEQ ID NO: 4 shows the amino acid sequence of NRSF/REST residues 39-55.
<SEQ ID NO: 5>
   SEQ ID NO: 5 shows the amino acid sequence of NRSF/REST residues 38-57.
<SEQ ID NO: 6>
   SEQ ID NO: 6 shows the amino acid sequence of NRSF/REST residues 64-80.
<SEQ ID NO: 7>
   SEQ ID NO: 7 shows the amino acid sequence of NRSF/REST residues 68-83.
<SEQ ID NO: 8>
   SEQ ID NO: 8 shows the amino acid sequence of NRSF/REST residues 42-56 to which Lys-Lys-Lys-Lys and Trp are added at the C-terminus.
<SEQ ID NO: 9>
   SEQ ID NO: 9 shows the amino acid sequence of NRSF/REST residues 43-56.
<SEQ ID NO: 10>
   SEQ ID NO: 10 shows the amino acid sequence of N-CoR residues 1834-1847.
<SEQ ID NO: 11>
   SEQ ID NO: 11 shows the amino acid sequence of Mad residues 6-21.
<SEQ ID NO: 12>
   SEQ ID NO: 12 shows the SID consensus sequence binding to mSin3B PAH2 domain.
<SEQ ID NO: 13>
   SEQ ID NO: 13 shows the SID consensus sequence binding to mSin3A PAH2 domain.
<SEQ ID NO: 14>
   SEQ ID NO: 14 shows the DNA sequence of NRSF/REST (a.a. 43-83) fragment inserted into the BamHI-EcoRI site of pGEX-2T.
<SEQ ID NO: 15>
   SEQ ID NO: 15 shows the DNA sequence of mSin3B PAH1 domain (a.a. 28-107) fragment inserted into the NdeI-BamHI site of pET-28a.
<SEQ ID NO: 16>
   SEQ ID NO: 16 shows the amino acid sequence of mSin3B PAH1 domain (a.a. 28-107).
<SEQ ID NO: 17>
   SEQ ID NO: 17 shows the amino acid sequence of N-CoR residues 1829-1847.

## Claims

1. A peptide of 8 to 50 amino acids, comprising the following amino acid sequence:
Leu-Ile-Met-Leu-Ala-Asn-Val-Ala (SEQ ID NO: 1) (I)

2. The peptide according to claim 1, wherein Gln is added to the N terminus of sequence (I) and/or Leu is added to the C terminus of sequence (I).

3. The peptide according to claim 1, wherein Ala-Pro-Gln is added to the N terminus of sequence (I) and/or Leu-Thr is added to the C terminus of sequence (I).

4. The peptide according to any one of claims 1 to 3, which is capable of binding to the PAH1 domain of Sin3.

5. The peptide according to any one of claims 1 to 4, which contains an amino acid sequence identical to the amino acid sequence of a specific region of neural restrictive silencer factor NRSF/REST.

6. The peptide according to claim 5, which contains the amino acid sequence as shown in any one of SEQ ID NO: 3, 4 or 5.

7. The peptide according to any one of claims 1 to 6, wherein basic amino acid residues are added to its C terminus.

8. The peptide according to claim 7, wherein an amino acid residue with an aromatic ring is further added.

9. The peptide according to claim 8, which contains the amino acid sequence as shown in SEQ ID NO: 8.

10. A peptide of 8 to 50 amino acids, comprising the following amino acid sequence:
Φ¹-X¹-X²-Φ²-A¹-X³-A²-Ala (SEQ ID NO: 2) (II)
where Φ¹ and Φ² each independently represent a bulky hydrophobic amino acid residue; X¹, X² and X³ each independently represent any amino acid residue other than Pro; and A¹ and A² are each independently represent Ala or Val.

11. The peptide according to claim 10, which is capable of binding to the PAH1 domain of Sin3.

12. The peptide according to claim 10 or 11, which contains an amino acid sequence identical to the amino acid sequence of a specific region of neural restrictive silencer factor NRSF/REST or transcriptional co-repressor N-CoR.

13. The peptide according to claim 12, which contains the amino acid sequence as shown in SEQ ID NO: 3, 4, 5 or 10.

14. The peptide according to any one of claims 10 to 13, wherein basic amino acid residues are added to its C terminus.

15. The peptide according to claim 14, wherein an amino acid residue with an aromatic ring is further added.

16. The peptide according to claim 15, which contains the amino acid sequence as shown in SEQ ID NO: 8.

17. A complex formed by the peptide according to any one of claims 1 to 16 and the PAH1 domain of Sin3.

18. A method of screening for substances effective in preventing and/or treating neurological diseases, comprising determining whether or not a test substance affects the interaction between the peptide according to any one of claims 1 to 16 and the PAH1 domain of Sin3.

19. A method of detecting neurological diseases, comprising using the peptide according to any one of claims 1 to 16 or an antibody thereto.
